# EUROPÄISCHE PATENTSCHRIFT

(11) **EP 4 184 428 B1**
(45) Veröffentlichungstag und Bekanntmachung des Hinweises auf die Patenterteilung: **08.05.2024**
(21) Anmeldenummer: 22208675.3
(22) Anmeldetag: 21.11.2022
(51) Int. Cl.: G06T 5/00, G06T 5/50, G06T 7/246, A61B 1/04, G06V 10/143, G06T 5/73, A61B 1/00

(54) **BILDGEBENDES VERFAHREN ZUM ABBILDEN EINER SZENE UND SYSTEM**
IMAGING METHOD FOR IMAGING A SCENE AND SYSTEM
PROCÉDÉ ET SYSTÈME D'IMAGERIE POUR IMAGER UNE SCÈNE

(30) Priorität: 22.11.2021 DE 102021130529
(43) Veröffentlichungstag der Anmeldung: 24.05.2023
(73) Patentinhaber: Karl Storz SE & Co. KG, 78532 Tuttlingen (DE)
(72) Erfinder: Steinke, Bernd, 78532 Tuttlingen (DE)
(74) Vertreter: Witte, Weller & Partner Patentanwälte mbB

(56) Entgegenhaltungen:
- EP-B1- 2 750 585
- WO-A1-2012/083968
- US-A1- 2012 013 773

## Beschreibung

Die Erfindung betrifft ein bildgebendes Verfahren zum Abbilden einer Szene während einer Veränderung eines Sichtfelds eines Bildsensors relativ zu der Szene. Die Erfindung betrifft ferner ein System zum Abbilden einer Szene während einer Veränderung eines Sichtfelds eines Bildsensors relativ zu der Szene.

Wie sich aus den nachfolgenden Erläuterungen ergibt, bietet die Erfindung Vorteile bei Anwendungen, bei denen Bildinformationen aus zwei verschiedenen Spektralbereichen verarbeitet werden sollen. Für die nachfolgenden Erläuterungen soll es sich bei diesen Spektralbereichen um den Weißlichtbereich und den Nahinfrarotbereich handeln. Die Erfindung ist aber nicht auf diese Spektralbereiche beschränkt. Zudem soll beispielhaft auf Bilder eingegangen werden, die in der Endoskopie gewonnen werden. Die Erfindung ist aber nicht auf diese Art von Bildern beschränkt.

Bildinformationen aus zwei verschiedenen Spektralbereichen entstehen beispielsweise bei der Bildgebung unter Zuhilfenahme der Nahinfrarotfluoreszenz, siehe beispielsweise https://www.karlstorz.com/ro/en/nir-icg-near-infrared-fluorescence.htm. Die bisherigen Fluoreszenz-Bildgebungsprodukte benötigen sehr leistungskräftige Lichtquellen im Weißlichtbereich und insbesondere für das Nahinfrarot(NIR)-Fluoreszenzlicht.

Dies ist bedingt durch mehrere Faktoren, so zum Beispiel Durchsatzbeschränkungen des Systems für Fluoreszenzlicht. Bei alternierendem Betrieb von Weiß- und Fluoreszenzlicht erhält man weniger Frames im jeweiligen Strom von Weiß- bzw. Fluoreszenzlichtbildern. Zudem können kürzere Belichtungszeiten erforderlich sein, um eine bestimmte Bildwiederholungsrate zu erzielen. Schließlich sind bei der Verwendung eines Rolling Shutter Wartezeiten erforderlich, um fehlerhafte Frames zu vermeiden, die zum Beispiel partielle Überlagerungen von Weiß- und Fluoreszenzlicht zeigen würden.

Die hat zur Folge, dass Fluoreszenzaufnahmen weniger Kontrast haben bzw. verwaschen aussehen. Schwächere Fluoreszenzeffekte sind dabei weniger genau erkennbar. Ein resultierendes NIR-Bild zeigt einen grünen Schimmer und hat deutlich weniger Kontrast und Details als ein Weißlichtbild. Das NIR-Bild enthält aber Informationen, die gerade im medizinischen Bereich eine große Rolle spielen und in einem Weißlichtbild nicht zu erkennen sind.

Die traditionelle und oft genutzte Lösung zur Erzeugung von NIR-Licht sind Hochleistungs-Xenon-Lampen. Diese haben allerdings einige Nachteile, wie zum Beispiel eine relativ kurze Lebensdauer mit früher Degeneration und erheblicher Lautstärke aufgrund der prinzipbedingt erforderlichen leistungsstarken Lüftung. Der Grund dafür ist, dass zur Bereitstellung des Fluoreszenzlichts nur der NIR-Lichtanteil des breitbandigen Xenon-Lichts genutzt wird. Der daneben erzeugte leistungsstarke Weißlicht-Anteil wird ausgefiltert und muss als Wärme abgeführt werden. Zudem erzeugen die Xenon-Lampen bereits selbst relevante Abwärme.

Daher kommen heute verstärkt LEDs zum Einsatz, die eine geringere Abwärme, ein verringertes Lüftergeräusch und eine erhöhte Lampenlebensdauer bieten. Die begrenzte IR Lichtintensität der LED benötigt allerdings eine im Vergleich größere Verstärkung des Bildsignals, zum Beispiel mittels einer Automatic Gain Control (AGC). Die Verstärkung hat allerdings den Nachteil eines verstärkten Rauschens und damit einer gewissen Unscharfe und ggf. verringertem Kontrast im Fluoreszenzkanal um dieses Rauschen heraus zu mitteln. Zwar kann versucht werden, diesem Effekt mit einer längeren Belichtung entgegenzuwirken. Dies führt allerdings schon bei einem leichten Verwackeln zu qualitativ beschränkten Bildern.

Schließlich kommen auch laserbasierte Lösungen zum Einsatz. Da hier jedoch Laserschutzbrillen erforderlich sind, werden Laser in aller Regel nur eingesetzt, wenn andere Möglichkeiten nicht die gewünschten Ergebnisse liefern können.

Um ein gutes Ergebnis bei der bildlichen Darstellung zu erzielen, sind ein erhöhter Detaillierungsgrad, weniger Bildrauschen und ein höherer Kontrast gewünscht. Ganz konkret kann eine solche gute bildliche Darstellung die ärztliche Interpretation von endoskopischen Abbildungen erleichtern, zum Beispiel von regional unzureichenden Perfusionsbereichen. So kann insbesondere bei scharfen, mit gutem Dynamikumfang sicher erkennbaren Grenzen zuverlässig zwischen gut perfundierten Bereichen und unzureichend mit der Blutversorgung verbundenen Gewebeteilen unterschieden werden. Dies gilt ebenso bei der trennscharfen Erkennbarkeit von karzionösem Gewebebereichen im Gegensatz zu gesundem Gewebe.

US 2012/013773 A1 betrifft eine Bildverarbeitungsvorrichtung mit einem Bewegungsvektor-Rechner und einer Ausrichtungsverarbeitungseinheit. Der Bewegungsvektorrechner berechnet Bewegungsvektorinformationen zwischen einem Fluoreszenzbild eines beobachteten Bereichs auf der Grundlage von Fluoreszenz, die von dem mit Anregungslicht bestrahlten beobachteten Bereich erzeugt wird, und einem Auflichtbild des beobachteten Bereichs auf der Grundlage des von dem beobachteten Bereich reflektierten Lichts. Die Ausrichtungsverarbeitungseinheit korrigiert die Fehlausrichtung eines Objekts zwischen dem Fluoreszenzbild und dem Auflichtbild des beobachteten Bereichs auf der Grundlage der Bewegungsvektorinformationen.

EP 2 750 585 B1 betrifft ein Verfahren zur Identifizierung von Karies, das zumindest teilweise auf Datenverarbeitungshardware ausgeführt wird, bei dem eine Vielzahl von Blöcken digitaler Zahnbilder erfasst wird, wobei jeder Block von Zahnbildern ein Fluoreszenzbild und ein oder mehrere Reflexionsbilder umfasst. Mindestens ein registriertes Fluoreszenzbild wird aus dem Fluoreszenzbild und dem einen oder den mehreren Reflexionsbildern erzeugt. Aus jedem der ein oder mehreren Reflexionsbilder und dem mindestens einen registrierten Fluoreszenzbild wird ein kombiniertes Bild erzeugt.

WO 2012/083968 A1 betrifft ein Verfahren zur Kompensation von Bewegungsunschärfe bei der Durchführung einer 3D-Abtastung von mindestens einem Teil eines Objekts mittels eines 3D-Scanners, wobei die Bewegungsunschärfe auftritt, weil der Scanner und das Objekt während der Abtastung relativ zueinander bewegt werden, und wobei die Kompensation der Bewegungsunschärfe Folgendes umfasst: Bestimmen, ob während der Erfassung der Folge von Fokusebenenbildern eine Relativbewegung zwischen dem Scanner und dem Objekt vorliegt; -wenn eine Relativbewegung bestimmt wird, Durchführen einer Bewegungskompensation auf der Grundlage der bestimmten Bewegung; und Erzeugen einer 3D-Oberfläche aus der Folge von Fokusebenenbildern.

Es ist eine Aufgabe der vorliegenden Erfindung ein verbessertes bildgebendes Verfahren und ein entsprechendes System zum Abbilden einer Szene während einer Veränderung eines Sichtfelds eines Bildsensors relativ zu der Szene aufzuzeigen.

Nach einem Aspekt wird die Aufgabe gelöst durch ein bildgebendes Verfahren zum Abbilden einer Szene nach Anspruch 1.

Im Rahmen der vorliegenden Erfindung hat der Erfinder erkannt, dass die herkömmlichen Wege zur Verbesserung der Bildqualität bei den zweiten Bildern, die hauptsächlich zweite Bildinformationen aus der Szene in einem unsichtbaren Lichtbereich aufweisen, an ihre Grenzen stoßen. Beispielsweise führen empfindlichere Bildchips für die Aufnahme der zweiten Bilder zu erheblich höheren Kosten. Eine Verlängerung des Belichtungszeit für ein zweites Bild führt aufgrund der Veränderung des Sichtfelds des Bildsensors zu einem Verwischen von Konturen. Eine Verstärkung der Lichtintensität hat gerade im Bereich der Fluoreszenzbildgebung nur eine begrenzte Wirkung, da nicht ein reflektiertes Licht erfasst wird, sondern eine durch das Licht ausgelöste Fluoreszenzantwort. Die Begriffe "sichtbar" und "unsichtbar" beziehen sich dabei auf das menschliche Sehvermögen, wobei "sichtbar" ein Spektrum beschreibt, für welches das menschliche Auge empfindlich ist, und "unsichtbar" ein Spektrum beschreibt, für welches das menschliche Auge unempfindlich ist.

Im Rahmen der vorliegenden Erfindung hat der Erfinder ferner erkannt, dass sich auch keine zufriedenstellende Lösung ergibt, wenn man mehrere zweite Bilder direkt überlagert, da dies aufgrund der Veränderung des Sichtfelds des Bildsensors im Ergebnis einem Verwischen ähnelt. Dabei hat der Erfinder auch eine direkte Registrierung (https://de.wikipedia.org/wiki/Bildregistrierung bzw. https://en.wikipedia.org/wiki/lmage_registration) der zweiten Bilder in Erwägung gezogen, was sich jedoch nicht in allen Situationen als praktikabel erwiesen hat, insbesondere, wenn die zweiten Bilder, zum Beispiel aufgrund eines geringen Kontrasts, eine Registrierung nicht oder nur ungenau ermöglichen.

Eine Besonderheit der aufgezeigten Lösung ist, dass eine Veränderung des Sichtfelds des Bildsensors anhand von Bildinformationen aus Bildern ermittelt wird, die hauptsächlich Bildinformationen im sichtbaren Lichtbereich aufweisen. Diese Bildinformationen haben in der Praxis hinreichend klar erkennbare Merkmale, mit Hilfe derer sich die Veränderung ermitteln lässt. Die Veränderung kann dabei aus zwei unmittelbar aufeinanderfolgenden Bildern mit Bildinformationen im sichtbaren Lichtbereich ermittelt werden oder auch Bildern, die zeitlich aufeinanderfolgen, ohne aber unmittelbar aufeinanderzufolgen.

Nun ist es ferner bekannt, zum Beispiel aufgrund der Ansteuerung einer bildgebenden Vorrichtung oder der verwendeten bildgebenden Technologie, wann die Bilder aufgenommen werden, die hauptsächlich Bildinformationen in einem unsichtbaren Lichtbereich aufweisen. Nimmt man nun an, dass die Veränderung des Sichtfelds des Bildsensors zwischen den Bildern, die hauptsächlich Bildinformationen im sichtbaren Lichtbereich aufweisen, zumindest im Wesentlichen gleichförmig ist, so kann man berechnen, wie weit die Veränderung bei der Aufnahme der Bilder, die hauptsächlich Bildinformationen in einem unsichtbaren Lichtbereich aufweisen, fortgeschritten war. Die Veränderung kann dabei insbesondere eines oder mehrere Elemente aus der Gruppe Translation, Rotation, Kippen, Veränderung einer optischen Brennweite oder Veränderung einer digitalen Vergrößerung aufweisen.

Wenn man beispielsweise annimmt, dass eine Veränderung A zwischen einem ersten Zeitpunkt t1, an dem das erste Bild aufgenommen wird, und einem dritten Zeitpunkt t3, an dem das dritte Bild aufgenommen wird, T = t3 - t1 beträgt, und ein zweites Bild zu einem Zeitpunkt t2 = 0,5 T aufgenommen wird, so beträgt die Veränderung zwischen dem ersten Bild und dem zweiten Bild 0,5 A und auch zwischen dem zweiten Bild und dem dritten Bild 0,5 A.

Bei einem anderen Beispiel werden zwei zweite Bilder zu einem Zeitpunkt t2 = 0,4 T und t2' = 0,6 T aufgenommen. Hier kann man sich nun in besonderer Weise zu Nutze machen, dass die Veränderung zwischen dem ersten zweiten Bild und dem zweiten zweiten Bild T = t2' - t2 = 0,2 T beträgt, so dass die Veränderung zwischen den zweiten Bildern zu 0,2 A berechnet werden kann.

Vertieft man dieses Beispiel erkennt man, dass die zweiten Bilder nun registriert werden können, weil die anteilige Veränderung zwischen den zweiten Bildern bekannt ist. Es ist also nicht erforderlich, die Veränderung aus den zweiten Bildern selbst zu ermitteln. Dies kann zwar zusätzlich erfolgen, um die Genauigkeit zu erhöhen oder eine Prüfung auf Plausibilität zu durchzuführen. Es ist aber nicht zwingend erforderlich, die zweiten Bilder selbst bzgl. einer Veränderung zu untersuchen. Die Kenntnis der Veränderung in den korrespondierenden Bildern, die hauptsächlich Bildinformationen im sichtbaren Lichtbereich aufweisen, ermöglicht es, die anteilige Veränderung bei den zweiten Bildern zu ermitteln.

Ferner können auch die zweiten Bilder mit dem ersten und/oder dem dritten Bild registriert werden, da bei dem genannten Beispiel bekannt ist, dass die Veränderung zwischen dem ersten Bild und dem ersten zweiten Bild 0,4 A beträgt und auch die Veränderung zwischen dem zweiten zweiten Bild und dem dritten Bild 0,4 A beträgt. So kann beispielsweise das erste zweite Bild mit dem zweiten zweiten Bild registriert werden, oder umgekehrt, und das Ergebnisbild mit dem ersten und/oder dem dritten Bild registriert werden.

Für die Registrierung der zweiten Bilder wird jedem zweiten Bild ein jeweiliger zweiter Zeitpunkt zugeordnet. Dies kann insbesondere der jeweilige zweite Zeitpunkt sein, an dem das jeweilige zweite Bild aufgenommen wurde. Bei langsamen Veränderungen und insbesondere wenn mehrere Bilder registriert werden sollen, die hauptsächlich Bildinformationen im unsichtbaren Lichtbereich aufweisen, aber zwischen unterschiedlichen Bildern liegen, die hauptsächlich Bildinformationen im sichtbaren Lichtbereich aufweisen, kann es ausreichend sein, den zweiten Bildern, die zwischen denselben Bildern liegen, die hauptsächlich Bildinformationen im sichtbaren Lichtbereich aufweisen, einen bestimmten zweiten Zeitpunkt zuzuordnen, ohne jedem zweiten Bild einen individuellen zweiten Zeitpunkt zuzuordnen.

Es sei darauf hingewiesen, dass nicht alle Bilder, die hauptsächlich Bildinformationen im unsichtbaren Lichtbereich aufweisen, verarbeitet werden müssen. Vielmehr kann eine Auswahl getroffen werden, so dass nur eine Teilmenge der aufgenommenen Bilder die zweiten Bilder darstellt, die in Übereinstimmung gebracht wird. Die zweiten Bilder für die Verarbeitung können demnach auch eine Teilmenge aller zweiten Bilder sein. Schließlich müssen auch nicht alle zweiten Zeitpunkte unterschiedlich sein.

Die vorgeschlagene Lösung ist die Optimierung sowohl des Weißlichtbildes als auch insbesondere des Fluoreszenzlichtbildes. Da beide alternierend in definiertem zeitlichem Verlauf korrelieren erweitert die Ausnutzung dieser Besonderheit die technischen Aufgaben die Effekte dieses bi-spektralen zeitlichen Versatzes konstruktiv zur Bildverbesserung zu nutzen. Es wird also nicht eine triviale "Brüte Force"-Verstärkung der (LED-)Lichtquelle oder der Kamerasensoren vorgeschlagen, sondern die innovative Nutzung von Videobearbeitungsalgorithmen für Fluoreszenzlichtaufnahmen inklusive einer Korrektur unter Nutzung des sichtbaren Lichtbereichs und über mehrere Frames.

Es gibt nun die spezifische Verwendung von abwechselnd sichtbarem Licht und unsichtbarem Licht, insbesondere NIR-Licht bzw. Fluoreszenzlicht, bei nun zwei getrennten aber orts- und veränderungs-übergreifend korrelierten Kanälen. Es wird ja dieselbe Szene nur in unterschiedlichen Spektren registriert. Der zeitliche Versatz ist dabei geringfügig. Die schwächere unsichtbare Information, insbesondere NIR-Information, wird bevorzugt nicht zur Verfolgung einer Veränderung bei der Aufnahme der Bilder verwendet, da der Erfinder erkannt hat wird, dass dies alleine unnötig ungenau, da zu schwach, unscharf und zu wenig detailliert wäre.

Die Bewegungskorrelation zum Weißlicht wird nun durch extrapolierte Bewegungskompensationsdaten vom sichtbaren Licht zum unsichtbaren Licht, insbesondere Fluoreszenzlicht, übertragen und wird für die nötige Bildoptimierung im unsichtbaren Licht genutzt. Die Bewegungskompensation hat sich in der Praxis der Endoskopie als sehr effektiv herausgestellt, da Bewegungen aufgrund der trägeren Masse des endoskopischen Systems und ggf. der zusätzlichen Fixierung an Halterungen tatsächlich eine hinreichende Gleichförmigkeit haben. Die Bildoptimierung wird bewegungskompensiert über mehrere vergangene Frames basierend auf der Veränderung der Bilder im sichtbaren Lichtbereich, insbesondere beschrieben durch Weißlichtvektoren, auf die Bilder im unsichtbaren Lichtbereich, insbesondere NIR-Bilder, angewendet.

Es sei darauf hingewiesen, dass die erste Beleuchtung und die zweite Beleuchtung durch verschiedene Lichtquellen bereitgestellt werden können, aber auch mit einer Lichtquelle. Im letztgenannten Fall kann zum Beispiel ein schaltbares Filter verwendet werden, welches für Weißlich kaum durchlässig ist oder hauptsächlich nur für die Anregungswellenlänge der Fluoreszenz durchlässig ist. Es ist aber auch möglich auf ein solches Filter für die Lichtquelle zu verzichten, wenn zwei Bildsensoren für die Bildaufnahme verwendet werden, von denen einer im Wesentlichen für sichtbares Licht empfindlich ist, während der andere im Wesentlichen für unsichtbares Licht empfindlich ist. Grundsätzlich kann aber auch nur ein Bildsensor eingesetzt werden, wenn diesem das Filter vorgeschaltet ist, so dass dem Bildsensor im Wesentlichen entweder sichtbares Licht oder unsichtbares Licht zugeführt wird.

Dadurch ist die Aufgabe vollständig gelöst.

Bei einer bevorzugten Ausgestaltung weist das Verfahren des Weiteren folgende Schritte auf:
i) nach Schritt b) und vor Schritt c), Erfassen eines vierten Bilds in der Szene bei der ersten Beleuchtung der Szene zu einem vierten Zeitpunkt, wobei das vierte Bild hauptsächlich vierte Bildinformationen aus der Szene in dem sichtbaren Lichtbereich aufweist,
j) nach Schritt i) und vor Schritt c), Erfassen einer Mehrzahl von fünften Bildern in der Szene bei der zweiten Beleuchtung der Szene zu einem jeweiligen fünften Zeitpunkt, wobei die fünften Bilder hauptsächlich fünfte Bildinformationen aus der Szene in dem unsichtbaren Lichtbereich aufweisen,
k) nach Schritt e) und vor Schritt g), Registrieren der fünften Bilder unter Berücksichtigung mindestens einer dritten Veränderung, die sich als zweiter Anteil der ersten Veränderung ergibt, wobei der zweite Anteil eine dritte Zeitdifferenz der fünften Zeitpunkte von zwei zu registrierenden fünften Bilden im Verhältnis zu der zweiten Zeitdifferenz zwischen dem dritten Zeitpunkt und dem ersten Zeitpunkt ist, und
wobei im Schritt g) die registrierten zweiten Bilder und die registrierten fünften Bilder verarbeitet werden, um ein Ergebnisbild zu erhalten.

Diese Ausgestaltung ermöglicht es noch weitergehend Bilder, die hauptsächlich Bildinformationen in einem unsichtbaren Lichtbereich aufweisen, miteinander zu registrieren. Dabei ist es auch möglich, Bilder rollierend zu registrieren, z.B. zunächst ein erstes zweites Bild mit einem zweiten zweiten Bild, dann das zweite zweite Bild mit einem ersten fünften Bild, dann das erste fünfte Bild mit einem zweiten fünften Bild, und so weiter.

Bei einer bevorzugten Ausgestaltung wird
der Schritt d) stattdessen wie folgt ausgeführt wird: Ermitteln mindestens eines ersten Referenzmerkmals in der Szene, das im ersten und im vierten Bild abgebildet ist, und mindestens eines zweiten Referenzmerkmals in der Szene, das im vierten und im dritten Bild abgebildet ist,
der Schritt e) stattdessen wie folgt ausgeführt wird: Ermitteln einer ersten Veränderung des Sichtfelds anhand des mindestens einen ersten Referenzmerkmals und einer weiteren Veränderung des Sichtfelds anhand des mindestens einen zweiten Referenzmerkmals, und
der Schritt f) stattdessen wie folgt ausgeführt wird: Registrieren der zweiten Bilder unter Berücksichtigung mindestens einer zweiten Veränderung, die sich als erster Anteil der ersten Veränderung ergibt, wobei der erste Anteil eine erste Zeitdifferenz der zweiten Zeitpunkte von zwei zu registrierenden zweiten Bilden im Verhältnis zu einer zweiten Zeitdifferenz zwischen dem vierten Zeitpunkt und dem ersten Zeitpunkt ist,
der Schritt k) stattdessen wie folgt ausgeführt wird: Registrieren der fünften Bilder unter Berücksichtigung mindestens einer dritten Veränderung, die sich als zweiter Anteil der weiteren Veränderung ergibt, wobei der zweite Anteil eine dritte Zeitdifferenz der fünften Zeitpunkte von zwei zu registrierenden fünften Bildern im Verhältnis zu einer vierten Zeitdifferenz zwischen dem dritten Zeitpunkt und dem vierten Zeitpunkt ist.

Diese Ausgestaltung ermöglicht es das vierte Bild als Stützbild für die Registrierung der zweiten und fünften Bilder zu verwenden. Dies kann die Genauigkeit bei der Registrierung erhöhen.

Bei einer bevorzugten Ausgestaltung ist eine erste Intensität des ersten Bilds und des dritten Bilds jeweils größer als eine zweite Intensität jedes der zweiten Bilder.

Dabei kann die zweite Intensität insbesondere höchstens 50%, 33%, 25%, 20%, 15% oder 10% der ersten Intensität betragen. Die Intensität eines Bilds kann insbesondere als Durchschnitt oder Median aller Pixel ermittelt werden.

Bei einer bevorzugten Ausgestaltung weisen die zweiten Bilder hauptsächlich zweite Bildinformationen aus der Szene in einem Nahinfrarotbereich auf.

Diese Ausgestaltung ist besonders geeignet für die Anwendung auf dem Gebiet der Fluoreszenzbildgebung.

Bei einer bevorzugten Ausgestaltung werden die zweiten Bilder in Übereinstimmung mit einem Referenzbild gebracht.

Ein solches Referenzbild kann damit sowohl ein Bild aus dem sichtbaren Lichtbereich als auch aus dem unsichtbaren Lichtbereich sein. Während es im erstgenannten Fall gewünscht ist, die Bilder aus dem unsichtbaren Lichtbereich zusammen mit den Bildern aus dem sichtbaren Lichtbereich darzustellen, z.B. als Overlay, ist es im letztgenannten Fall gewünscht, ein separates Bild mit Bildinformationen aus dem unsichtbaren Lichtbereich darzustellen.

Bei einer bevorzugten Ausgestaltung weist das Verarbeiten der zweiten registrierten Bilder die Anwendung von Computational Photography auf.

Hier bieten sich vielfältige Möglichkeiten, die Bildinformationen aus dem unsichtbaren Lichtbereich mit höherer Qualität darzustellen, z.B. mit deutlicheren Konturen. Die Anwendung der Computational Photography, siehe z.B. https://en.wikipedia.org/wiki/Computational_photography, ist möglich, da die Bilder mit Bildinformationen aus dem unsichtbaren Lichtbereich registriert werden können, auch wenn deren Bildinformationen selbst eine übliche Registrierung nicht oder nicht hinreichend genau erlauben.

Ausführungsbeispiele der Erfindung sind in der Zeichnung dargestellt und werden in der nachfolgenden Beschreibung näher erläutert. Es zeigen:
- Fig. 1: eine erste Ausführungsform eines bildgebenden Verfahrens;
- Fig. 2: einen ersten zeitlichen Verlauf der Aufnahme von Bildern;
- Fig. 3: einen zweiten zeitlichen Verlauf der Aufnahme von Bildern;
- Fig. 4: einen dritten zeitlichen Verlauf der Aufnahme von Bildern;
- Fig. 5: eine zweite Ausführungsform eines bildgebenden Verfahrens;
- Fig. 6: eine dritte Ausführungsform eines bildgebenden Verfahrens;
- Fig. 7: einen vierten zeitlichen Verlauf der Aufnahme von Bildern;
- Fig. 8: einen fünften zeitlichen Verlauf der Aufnahme von Bildern;
- Fig. 9: eine erste Ausführungsform eines Systems zum Abbilden einer Szene; und
- Fig. 10: eine zweite Ausführungsform eines Systems zum Abbilden einer Szene.

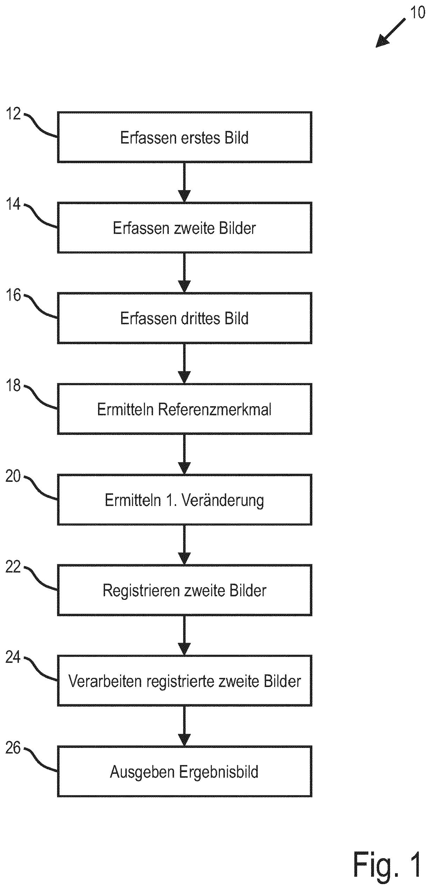

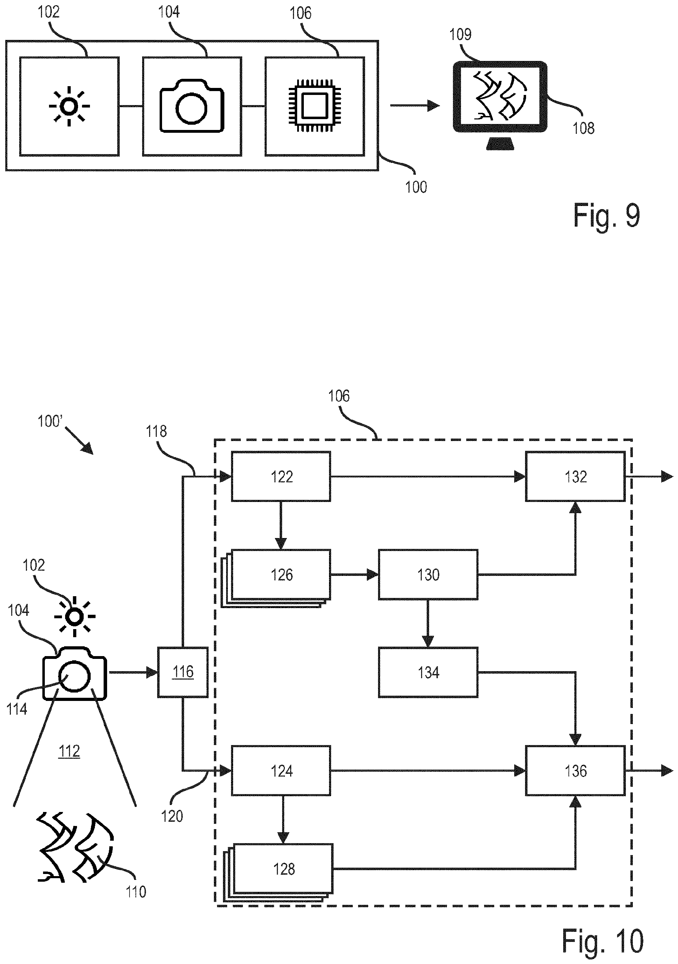

Fig. 1 zeigt eine erste Ausführungsform eines bildgebenden Verfahrens 10 zum Abbilden einer Szene 110 (siehe Fig. 10) während einer Veränderung eines Sichtfelds 112 (siehe Fig. 10) eines Bildsensors 114 (siehe Fig. 10) relativ zu der Szene 110. Die Schritte des Verfahrens 10 werden nachfolgend erläutert.

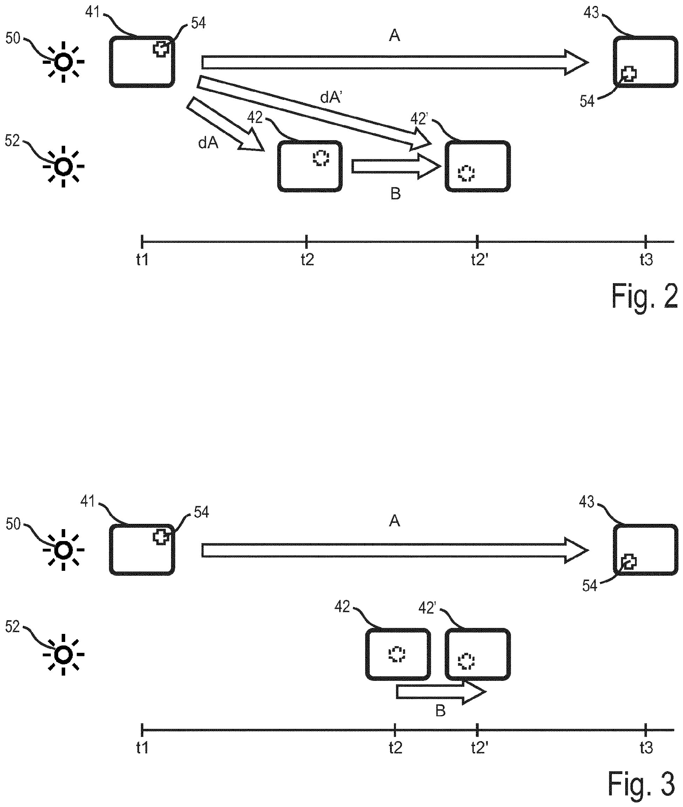

In einem Schritt 12 wird ein erstes Bild 41 (siehe Fig. 2) in der Szene bei einer ersten Beleuchtung 50 (siehe Fig. 2) der Szene 110 zu einem ersten Zeitpunkt t1 (siehe Fig. 2) erfasst, wobei das erste Bild 41 hauptsächlich erste Bildinformationen aus der Szene 110 in einem sichtbaren Lichtbereich aufweist.

In einem Schritt 14, der dem Schritt 12 folgt, wird eine Mehrzahl von zweiten Bildern 42, 42' (siehe Fig. 2) in der Szene 110 bei einer zweiten Beleuchtung 52 (siehe Fig. 2) der Szene 110 zu einem jeweiligen zweiten Zeitpunkt t2, t2' (siehe Fig. 2) erfasst, wobei die zweiten Bilder 42, 42' hauptsächlich zweite Bildinformationen aus der Szene in einem unsichtbaren Lichtbereich aufweisen.

In einem Schritt 16, der dem Schritt 14 folgt, wird ein drittes Bild 43 in der Szene 110 bei der ersten Beleuchtung 50 der Szene 110 zu einem dritten Zeitpunkt t3 (siehe Fig. 2), wobei das dritte Bild 43 hauptsächlich dritte Bildinformationen aus der Szene 110 in dem sichtbaren Lichtbereich aufweist.

In einem Schritt 18 wird mindestens ein Referenzmerkmal 54 (siehe Fig. 2) in der Szene 110 ermittelt, das im ersten und im dritten Bild 41, 43 abgebildet ist. Es ist in der Fig. 2 symbolisch dargestellt, dass das Referenzmerkmal 54 auch in den zweiten Bildern 42, 42' enthalten ist. Allerdings ist das Merkmal in den Bildern nur unscharf zu erkennen und eignet sich nicht für eine Registrierung der zweiter Bilder 42, 42' alleine anhand der in den zweiten Bildern 42, 42' enthaltenen Bildinformation.

In einem Schritt 20 wird eine Veränderung A des Sichtfelds 112 anhand des mindestens einen Referenzmerkmals 54 ermittelt. Bei dem ersten zeitlichen Verlauf der Aufnahme von Bildern gemäß Fig. 2 ist die Veränderung A einfach als Translation dargestellt. Wie bereits ausgeführt, kann die Veränderung aber insbesondere eines oder mehrere Elemente aus der Gruppe Translation, Rotation, Kippen, Veränderung einer optischen Brennweite oder Veränderung einer digitalen Vergrößerung aufweisen.

In einem Schritt 22 werden die zweiten Bilder 42, 42' unter Berücksichtigung mindestens einer zweiten Veränderung B registriert, die sich als erster Anteil der ersten Veränderung A ergibt, wobei der erste Anteil eine erste Zeitdifferenz der zweiten Zeitpunkte t2, t2' von zwei zu registrierenden zweiten Bilden 42, 42' im Verhältnis zu einer zweiten Zeitdifferenz zwischen dem dritten Zeitpunkt t3 und dem ersten Zeitpunkt t1 ist. Dies kann bei einer Ausführungsform als Formel wie folgt ausgedrückt werden: B = A * (t2' - t2) / (t3 - t1).

Eine andere Herangehensweise bei dieser Ausführungsform ist wie folgt: Für jedes zweite Bild 42, 42' der zweiten Bilder 42, 42' wird die Veränderung A des Sichtfelds 112 auf den zweiten Zeitpunkt t2, t2' interpoliert, der dem zweiten Bild 42, 42' zugeordnet ist, um eine Teilveränderung dA, dA' des Sichtfelds 112 für das zweite Bild 42, 42' zu erhalten. Diese Teilveränderung dA kann insbesondere berechnet werden als dA = A * (t2 - t1) / (t3 - t1) bzw. dA' = A * (t2' - t1) / (t3 - t1). Zudem kann die zweite Veränderung B zwischen den zweiten Bildern 42, 42' dann als B = dA' - dA berechnet werden. Auch hier ergibt sich bei dieser Ausführungsform dann B = A * (t2' - t2) / (t3 - t1).

In einem Schritt 24 werden die zweiten Bilder 42, 42' registriert, um die zweiten Bilder 42, 42' unter Berücksichtigung der zweiten Veränderung B, alternativ der jeweiligen erhaltenen Teilveränderungen dA, dA', in Übereinstimmung zu bringen und so registrierte zweite Bilder zu erhalten.

In einem Schritt 26 werden die registrierten zweiten Bilder verarbeitet, um ein Ergebnisbild 109 (siehe Fig. 9) zu erhalten. So können beispielsweise die Helligkeitswerte der registrierten zweiten Bilder addiert werden, um den Kontrast zu erhöhen. Im Stand der Technik wäre eine längere Belichtungsdauer zum Einsatz gekommen, was jedoch zu einem Verwischen von Konturen führt. Die Verarbeitung der registrierten zweiten Bilder führt hingegen zu einem besseren Ergebnisbild ohne die Konturen zu verwischen.

In einem Schritt 28 wird schließlich das Ergebnisbild ausgegeben, insbesondere auf einen Monitor 108 (siehe Fig. 9). Das Ergebnis kann wahlweise als solches ausgegeben werden oder im Overlay zusammen mit dem ersten und/oder dritten Bild 41, 43.

Fig. 2 zeigt einen ersten zeitlichen Verlauf der Aufnahme von Bildern. Hier wird zu einem ersten Zeitpunkt t1 ein erstes Bild 41 aufgenommen, zu einem ersten zweiten Zeitpunkt t2 ein erstes zweites Bild 42, zu einem zweiten zweiten Zeitpunkt t2' ein zweites zweites Bild 42' und zu einem dritten Zeitpunkt t3 ein drittes Bild 43. Es sei darauf hingewiesen, dass auch drei, vier, fünf oder mehr zweite Bilder 42 zwischen dem ersten Bild 41 und dem dritten Bild 43 angefertigt werden können.

Anhand der Bewegung des Referenzmerkmals 54 wird symbolisch dargestellt, wie sich das Sichtfeld 112 des Bildsensors 114 relativ zu der Szene 110 verändert. Zwischen dem ersten Bild 41 und dem dritten Bild 43 ergibt sich eine Veränderung A, zwischen dem ersten Bild 41 und dem ersten zweiten Bild 42 ergibt sich eine Veränderung dA, zwischen dem ersten Bild 41 und dem zweiten zweiten Bild 42' ergibt sich eine Veränderung dA' und zwischen dem ersten zweiten Bild 42 und dem zweiten zweiten Bild 42' ergibt sich eine zweite Veränderung B.

Fig. 3 zeigt einen zweiten zeitlichen Verlauf der Aufnahme von Bildern. Es gelten dieselben Ausführungen wie zur Fig. 2, wobei die symbolischen Pfeile für die Veränderungen dA und dA' zur besseren Übersicht weggelassen wurden. Es ist zu erkennen, dass die technische Lösung eine beliebige zeitliche Positionierung der zweiten Bilder 42, 42' unterstützt.

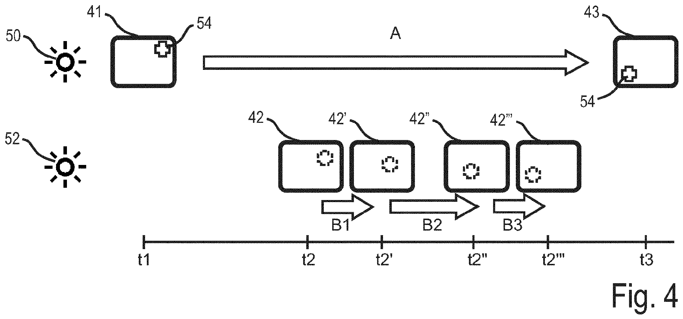

Fig. 4 zeigt einen dritten zeitlichen Verlauf der Aufnahme von Bildern. Es gelten dieselben Ausführungen wie zuvor. Im Unterschied zu den Figuren 2 und 3 werden hier vier zweite Bilder 42, 42', 42", 42‴ aufgenommen. Es können hier mehrere zweite Veränderungen ermittelt werden: B1 = A * (t2' - t2) / (t3 - t1), B2 = A * (t2" - t2') / (t3 - t1) und B3 = A * (t2‴ - t2") / (t3 -t1). Es besteht somit die Möglichkeit, alle vier zweiten Bilder 42, 42', 42", 42‴ miteinander zu registrieren.

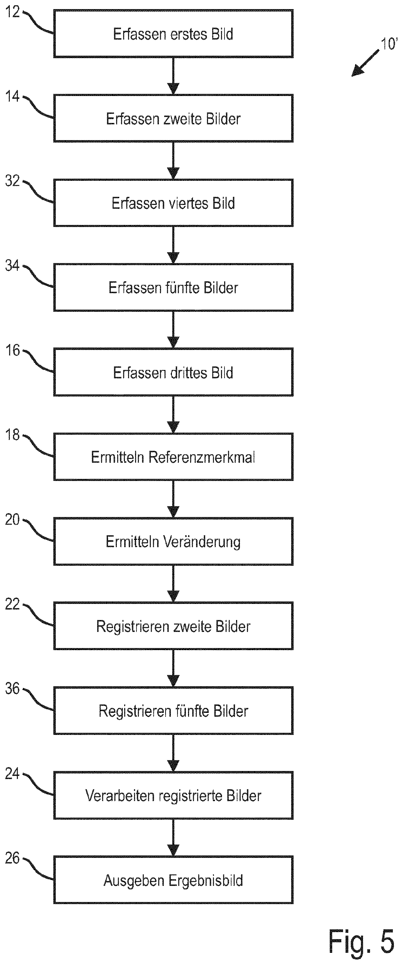

Fig. 5 zeigt eine zweite Ausführungsform eines bildgebenden Verfahrens 10' zum Abbilden einer Szene 110 während einer Veränderung eines Sichtfelds 112 eines Bildsensors 114 relativ zu der Szene 110. Die Schritte des Verfahrens 10' werden nachfolgend erläutert, wobei nur auf die Unterschiede zum Verfahren 10 aus Fig. 1 eingegangen wird.

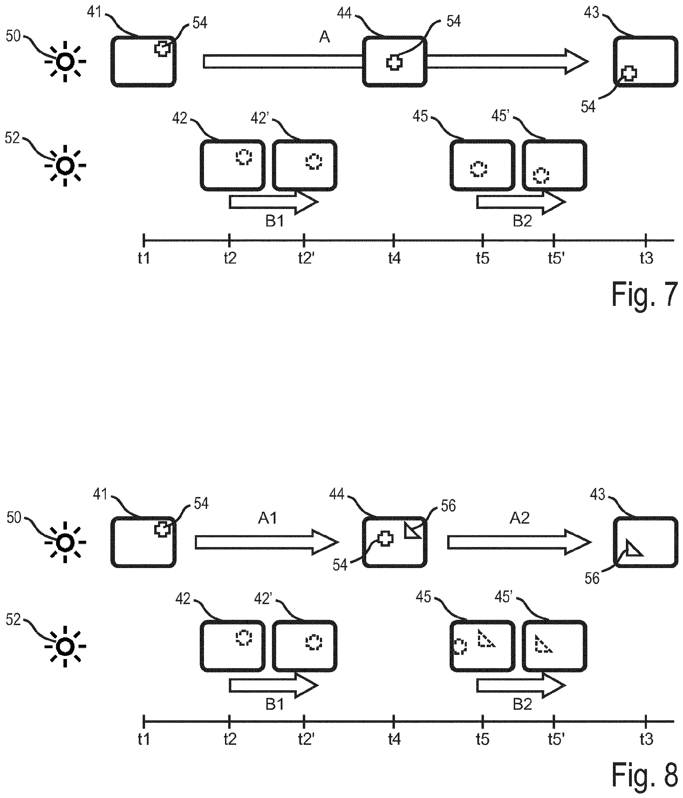

In einem Schritt 32 wird ein viertes Bild 44 (siehe Fig. 7) in der Szene 110 bei der ersten Beleuchtung 50 der Szene 110 zu einem vierten Zeitpunkt t4 erfasst, wobei das vierte Bild 44 hauptsächlich vierte Bildinformationen aus der Szene 110 in dem sichtbaren Lichtbereich aufweist.

In einem Schritt 34 wird eine Mehrzahl von fünften Bildern 45, 45' in der Szene 110 bei der zweiten Beleuchtung 52 der Szene 110 zu einem jeweiligen fünften Zeitpunkt t5 erfasst, wobei die fünften Bilder 45, 45' hauptsächlich fünfte Bildinformationen aus der Szene 110 in dem unsichtbaren Lichtbereich aufweisen.

In einem Schritt 36 werden die fünften Bilder 45, 45' unter Berücksichtigung mindestens einer dritten Veränderung B2 (siehe Fig. 7), die sich als zweiter Anteil der ersten Veränderung A ergibt, wobei der zweite Anteil eine dritte Zeitdifferenz der fünften Zeitpunkte t5, t5' von zwei zu registrierenden fünften Bilden 45, 45' im Verhältnis zu der zweiten Zeitdifferenz zwischen dem dritten Zeitpunkt t3 und dem ersten Zeitpunkt t1 ist. Dies kann bei einer Ausführungsform als Formel wie folgt ausgedrückt werden: B2 = A * (t5' - t5) / (t3 - t1).

Im Schritt 24 werden nun sowohl die registrierten zweiten Bilder und die registrierten fünften Bilder verarbeitet, um ein Ergebnisbild zu erhalten.

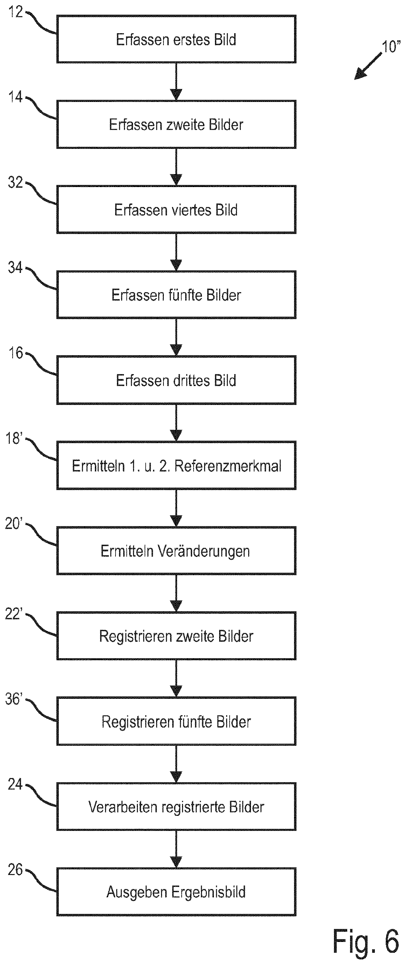

Fig. 6 zeigt eine dritte Ausführungsform eines bildgebenden Verfahrens 10" zum Abbilden einer Szene 110 während einer Veränderung eines Sichtfelds 112 eines Bildsensors 114 relativ zu der Szene 110. Die Schritte des Verfahrens 10" werden nachfolgend erläutert, wobei nur auf die Unterschiede zum Verfahren 10' aus Fig. 5 eingegangen wird.

Statt des Schritts 18 wird nun ein Schritt 18' ausgeführt, in dem mindestens eines erstes Referenzmerkmal 54 in der Szene 110 ermittelt wird, das im ersten und im vierten Bild 41, 44 abgebildet ist. Zudem wird mindestens ein zweites Referenzmerkmal 56 in der Szene 110, das im vierten und im dritten Bild 44, 43 abgebildet ist. Es ist möglich, dass das erste Referenzmerkmal 54 dasselbe ist wie das zweite Referenzmerkmal 56. Das erste Referenzmerkmal 54 kann vom zweiten Referenzmerkmal 56 aber auch verschieden sein.

Statt des Schritts 20 wird nun ein Schritt 20' ausgeführt, in dem eine erste Veränderung A1 des Sichtfelds 112 anhand des mindestens einen ersten Referenzmerkmals 54 ermittelt wird. Zudem wird eine weitere Veränderung A2 des Sichtfelds 112 anhand des mindestens einen zweiten Referenzmerkmals 56 ermittelt.

Statt des Schritts 22 wird nun ein Schritt 22' ausgeführt, in dem die zweiten Bilder 42, 42' unter Berücksichtigung mindestens einer zweiten Veränderung B1 registriert werden, die sich als erster Anteil der ersten Veränderung A1 ergibt, wobei der erste Anteil eine erste Zeitdifferenz der zweiten Zeitpunkte t2, t2' von zwei zu registrierenden zweiten Bilden 42, 42' im Verhältnis zu einer zweiten Zeitdifferenz zwischen dem vierten Zeitpunkt t4 und dem ersten Zeitpunkt t1 ist. Dies kann bei einer Ausführungsform als Formel wie folgt ausgedrückt werden: B1 = A * (t2' - t2) / (t4 - t1).

Statt des Schritts 36 wird nun ein Schritt 36' ausgeführt, in dem die fünften Bilder 45, 45' unter Berücksichtigung mindestens einer dritten Veränderung B2 registriert werden, die sich als zweiter Anteil der weiteren Veränderung A2 ergibt, wobei der zweite Anteil eine dritte Zeitdifferenz der fünften Zeitpunkte t5, t5' von zwei zu registrierenden fünften Bildern 45, 45' im Verhältnis zu einer vierten Zeitdifferenz zwischen dem dritten Zeitpunkt t3 und dem vierten Zeitpunkt t4 ist. Dies kann bei einer Ausführungsform als Formel wie folgt ausgedrückt werden: B2 = A * (t5' - t5) / (t3 - t4).

Fig. 7 zeigt einen vierten zeitlichen Verlauf der Aufnahme von Bildern. Es gelten dieselben Ausführungen wie zuvor. Hier wird zu einem ersten Zeitpunkt t1 ein erstes Bild 41 aufgenommen, zu einem ersten zweiten Zeitpunkt t2 ein erstes zweites Bild 42, zu einem zweiten zweiten Zeitpunkt t2' ein zweites zweites Bild 42', zu einem vierten Zeitpunkt t4 ein viertes Bild 44, zu einem ersten fünften Zeitpunkt t5 ein erstes fünftes Bild 45, zu einem zweiten fünften Zeitpunkt t5' ein zweites fünftes Bild 45' und zu einem dritten Zeitpunkt t3 ein drittes Bild 43. Es sei darauf hingewiesen, dass auch drei, vier, fünf oder mehr zweite Bilder 42 zwischen dem ersten Bild 41 und dem vierten Bild 44 angefertigt werden können und/oder drei, vier, fünf oder mehr fünfte Bilder 45 zwischen dem vierten Bild 44 und dem dritten Bild 43 angefertigt werden können.

Fig. 8 zeigt einen fünften zeitlichen Verlauf der Aufnahme von Bildern. Es gelten dieselben Ausführungen wie bei Fig. 7. Im Unterschied zur Fig. 7 wird das vierte Bild 44 hier als zusätzliches Stützbild verwendet, so dass die Registrierung der zweiten und fünften Bilder 42, 42', 45, 45' nicht auf der Basis der Veränderung A erfolgt, sondern getrennt erfolgt. Konkret erfolgt die Registrierung der zweiten Bilder 42, 42' auf Basis der ersten Veränderung A1 und die Registrierung der zweiten Bilder 42, 42' auf Basis der weiteren Veränderung A2.

Fig. 9 zeigt eine erste Ausführungsform eines Systems 100 zum Abbilden einer Szene 110 während einer Veränderung eines Sichtfelds 112 eines Bildsensors 114 relativ zu der Szene 110. Das System 100 weist mindestens eine Beleuchtungseinrichtung 102, mindestens eine bildgebende Vorrichtung 104 und eine Verarbeitungseinrichtung 106 auf. Die Verarbeitungseinrichtung 106 ist dafür ausgebildet, das System 100 zur Durchführung einer der zuvor beschriebenen Verfahren 10, 10', 10" zu veranlassen.

Fig. 10 zeigt eine zweite Ausführungsform eines Systems 100' zum Abbilden einer Szene 110 während einer Veränderung eines Sichtfelds 112 eines Bildsensors 114 relativ zu der Szene 110. Das System 100' weist mindestens eine Beleuchtungseinrichtung 102, mindestens eine bildgebende Vorrichtung 104 und eine Verarbeitungseinrichtung 106 auf.

Die bildgebende Vorrichtung 104 leitet den aufgenommenen Bilderstrom an eine Weiche 116, die einen Bilderstrom mit Bildern aus dem sichtbaren Lichtbereich in einen ersten Verarbeitungspfad 118 weiterleitet und einen Bilderstrom mit Bildern aus dem unsichtbaren Lichtbereich in einen zweiten Verarbeitungspfad 120 weiterleitet.

Die Bilderströme werden, jeweils entsprechend, in einer Segmentiervorrichtung 122 bzw. 124 in einzelne Bilder bzw. Frames unterteilt. Die Frames werden, jeweils entsprechend, in einer Speichervorrichtung 126 bzw. 128 für die weitere Verarbeitung gespeichert.

In einer Berechnungseinrichtung 130 wird die Veränderung A (oder die Veränderungen A1 und A2) aus den Frames im sichtbaren Lichtbereich berechnet oder geschätzt. Diese Information wird verwendet, um die Frames im sichtbaren Lichtbereich in einer Kompensationsvorrichtung 132 zu registrieren. Auf diese Weise kann beispielsweise einem Verwackeln entgegengewirkt werden.

Die ermittelte Veränderung A (oder die Veränderungen A1 und A2) wird verwendet, wie im Zusammenhang mit den vorherigen Figuren erläutert, um in einer Extra- und/oder Interpolationsvorrichtung 134 zu ermitteln, wie sich die ermittelte Veränderung A (oder die Veränderungen A1 und A2) auf die Bilder im unsichtbaren Lichtbereich ausgewirkt hat.

Diese Information, konkret die zweite Veränderung B (oder die Veränderungen B1 und B2) wird verwendet, um die Frames im unsichtbaren Lichtbereich in einer Kompensationsvorrichtung 136 zu registrieren. Auf diese Weise kann beispielsweise einem Verwackeln entgegengewirkt werden, auch wenn sich den Bildern im unsichtbaren Lichtbereich selbst keine hinreichende Information entnehmen lässt, um diese zu registrieren.

## Patentansprüche

1. Bildgebendes Verfahren (10) zum Abbilden einer Szene (110) während einer Veränderung eines Sichtfelds (112) eines Bildsensors (114) relativ zu der Szene (110), wobei das Verfahren (10) folgende Schritte aufweist:
a) Erfassen (12) eines ersten Bilds (41) in der Szene (110) bei einer ersten Beleuchtung (50) der Szene (110) zu einem ersten Zeitpunkt (t1), wobei das erste Bild (41) hauptsächlich erste Bildinformationen aus der Szene (110) in einem Spektralbereich des sichtbaren Lichts aufweist,
b) nach Schritt a), Erfassen (14) einer Mehrzahl von zweiten Bildern (42, 42') in der Szene (110) bei einer zweiten Beleuchtung (52) der Szene (110) zu einem jeweiligen zweiten Zeitpunkt (t2, t2'), wobei die zweiten Bilder (42, 42') hauptsächlich zweite Bildinformationen aus der Szene (110) in einem Spektralbereich des unsichtbaren Lichts aufweisen,
c) nach Schritt b), Erfassen (16) eines dritten Bilds (43) in der Szene (110) bei der ersten Beleuchtung (50) der Szene (110) zu einem dritten Zeitpunkt (t3), wobei das dritte Bild (43) hauptsächlich dritte Bildinformationen aus der Szene (110) in dem Spektralbereich des sichtbaren Lichts aufweist,
d) Ermitteln (18) mindestens eines Referenzmerkmals (54) in der Szene (110), das im ersten und im dritten Bild (41, 43) abgebildet ist,
e) Ermitteln (20) einer ersten Veränderung (A) des Sichtfelds anhand des mindestens einen Referenzmerkmals (54),
f) Registrieren (22) der zweiten Bilder unter Berücksichtigung mindestens einer zweiten Veränderung (B), die sich als erster Anteil der ersten Veränderung (A) ergibt, wobei der erste Anteil eine erste Zeitdifferenz der zweiten Zeitpunkte (t2, t2') von zwei zu registrierenden zweiten Bildern (42, 42') im Verhältnis zu einer zweiten Zeitdifferenz zwischen dem dritten Zeitpunkt (t3) und dem ersten Zeitpunkt (t1) ist,
g) Verarbeiten der registrierten zweiten Bilder, um ein Ergebnisbild zu erhalten, und
h) Ausgeben des Ergebnisbilds.

2. Verfahren (10') nach Anspruch 1, wobei das Verfahren des Weiteren folgende Schritte aufweist:
i) nach Schritt b) und vor Schritt c), Erfassen (32) eines vierten Bilds (44) in der Szene (110) bei der ersten Beleuchtung der Szene (110) zu einem vierten Zeitpunkt (t4), wobei das vierte Bild (44) hauptsächlich vierte Bildinformationen aus der Szene (110) in dem Spektralbereich des sichtbaren Lichts aufweist,
j) nach Schritt i) und vor Schritt c), Erfassen (34) einer Mehrzahl von fünften Bildern (45, 45') in der Szene (110) bei der zweiten Beleuchtung der Szene (110) zu einem jeweiligen fünften Zeitpunkt (t5, t5'), wobei die fünften Bilder (45, 45') hauptsächlich fünfte Bildinformationen aus der Szene (110) in dem Spektralbereich des unsichtbaren Lichts aufweisen,
k) nach Schritt e) und vor Schritt g), Registrieren (36) der fünften Bilder (45, 45') unter Berücksichtigung mindestens einer dritten Veränderung (B2), die sich als zweiter Anteil der ersten Veränderung (A) ergibt, wobei der zweite Anteil eine dritte Zeitdifferenz der fünften Zeitpunkte (t5, t5') von zwei zu registrierenden fünften Bildern (45, 45') im Verhältnis zu der zweiten Zeitdifferenz zwischen dem dritten Zeitpunkt (t3) und dem ersten Zeitpunkt ist (t1), und
wobei im Schritt g) die registrierten zweiten Bilder und die registrierten fünften Bilder verarbeitet werden, um ein Ergebnisbild zu erhalten.

3. Bildgebendes Verfahren (10) zum Abbilden einer Szene (110) während einer Veränderung eines Sichtfelds (112) eines Bildsensors (114) relativ zu der Szene (110), wobei das Verfahren (10) folgende Schritte aufweist:
a) Erfassen (12) eines ersten Bilds (41) in der Szene (110) bei einer ersten Beleuchtung (50) der Szene (110) zu einem ersten Zeitpunkt (t1), wobei das erste Bild (41) hauptsächlich erste Bildinformationen aus der Szene (110) in einem Spektralbereich des sichtbaren Lichts aufweist,
b) nach Schritt a), Erfassen (14) einer Mehrzahl von zweiten Bildern (42, 42') in der Szene (110) bei einer zweiten Beleuchtung (52) der Szene (110) zu einem jeweiligen zweiten Zeitpunkt (t2, t2`), wobei die zweiten Bilder (42, 42') hauptsächlich zweite Bildinformationen aus der Szene (110) in einem Spektralbereich des unsichtbaren Lichts aufweisen,
b1) nach Schritt b), Erfassen (32) eines vierten Bilds (44) in der Szene (110) bei der ersten Beleuchtung der Szene (110) zu einem vierten Zeitpunkt, wobei das vierte Bild (44) hauptsächlich vierte Bildinformationen aus der Szene (110) in dem Spektralbereich des sichtbaren Lichts aufweist,
b2) nach Schritt b1), Erfassen (34) einer Mehrzahl von fünften Bildern in der Szene (110) bei der zweiten Beleuchtung der Szene (110) zu einem jeweiligen fünften Zeitpunkt, wobei die fünften Bilder hauptsächlich fünfte Bildinformationen aus der Szene (110) in dem Spektralbereich des unsichtbaren Lichts aufweisen
c) nach Schritt b2), Erfassen (16) eines dritten Bilds (43) in der Szene (110) bei der ersten Beleuchtung (50) der Szene (110) zu einem dritten Zeitpunkt (t3), wobei das dritte Bild (43) hauptsächlich dritte Bildinformationen aus der Szene (110) in dem Spektralbereich des sichtbaren Lichts aufweist,
d) Ermitteln (18') mindestens eines ersten Referenzmerkmals (54) in der Szene (110), das im ersten und im vierten Bild (41, 44) abgebildet ist, und mindestens eines zweiten Referenzmerkmals (56) in der Szene (110), das im vierten (44) und im dritten Bild (43) abgebildet ist,
e) Ermitteln (20') einer ersten Veränderung (A1) des Sichtfelds (112) anhand des mindestens einen ersten Referenzmerkmals (54) und einer weiteren Veränderung (A2) des Sichtfelds (112) anhand des mindestens einen zweiten Referenzmerkmals (56),
f) Registrieren (22') der zweiten Bilder (42, 42') unter Berücksichtigung mindestens einer zweiten Veränderung (B1), die sich als erster Anteil der ersten Veränderung (A1) ergibt, wobei der erste Anteil eine erste Zeitdifferenz der zweiten Zeitpunkte (t2, t2') von zwei zu registrierenden zweiten Bildern (42, 42') im Verhältnis zu einer zweiten Zeitdifferenz zwischen dem vierten Zeitpunkt (t4) und dem ersten Zeitpunkt (t1) ist,
f1) Registrieren (36') der fünften Bilder (45, 45') unter Berücksichtigung mindestens einer dritten Veränderung (B2), die sich als zweiter Anteil der weiteren Veränderung (A2) ergibt, wobei der zweite Anteil eine dritte Zeitdifferenz der fünften Zeitpunkte (t5, t5') von zwei zu registrierenden fünften Bildern (45, 45') im Verhältnis zu einer vierten Zeitdifferenz zwischen dem dritten Zeitpunkt (t3) und dem vierten Zeitpunkt (t4) ist,
g) Verarbeiten der registrierten zweiten Bilder und der registrierten fünften Bilder, um ein Ergebnisbild zu erhalten, und
h) Ausgeben des Ergebnisbilds.

4. Verfahren (10, 10', 10") nach einem der vorhergehenden Ansprüche, wobei eine erste Intensität des ersten Bilds (41) und des dritten Bilds (43) jeweils größer ist als eine zweite Intensität jedes der zweiten Bilder (42, 42').

5. Verfahren (10, 10', 10") nach einem der vorhergehenden Ansprüche, wobei die zweiten Bilder (42, 42') hauptsächlich zweite Bildinformationen aus der Szene (110) in einem Nahinfrarotbereich aufweisen.

6. Verfahren (10, 10', 10") nach einem der vorhergehenden Ansprüche, wobei die zweiten Bilder (42, 42') in Übereinstimmung mit einem Referenzbild gebracht werden.

7. Verfahren (10, 10', 10") nach einem der vorhergehenden Ansprüche, wobei das Verarbeiten (24) der zweiten registrierten Bilder die Anwendung von Computational Photography aufweist.

8. System (100) zum Abbilden einer Szene (110) während einer Veränderung eines Sichtfelds (112) eines Bildsensors (114) relativ zu der Szene (110), das System (100) aufweisend:
mindestens eine Beleuchtungseinrichtung (102),
mindestens eine bildgebende Vorrichtung (104),
eine Verarbeitungseinrichtung (106), die dafür ausgebildet ist, das System (100) zur Durchführung eines Verfahrens nach einem der vorhergehenden Ansprüche zu veranlassen.

## Claims

1. An imaging method (10) for imaging a scene (110) during a change in a field of view (112) of an imaging sensor (114) relative to the scene (110), wherein the method (10) comprises the steps of:
a) capturing (12) a first image (41) in the scene (110) at a first illumination (50) of the scene (110) at a first point in time (t1), wherein the first image (41) comprises mainly first image information from the scene (110) in a spectral range of visible light,
b) after step a), capturing (14) a plurality of second images (42, 42') in the scene (110) at a second illumination (52) of the scene (110) at a respective second point in time (t2, t2'), wherein the second images (42, 42') comprise mainly second image information from the scene (110) in a spectral range of invisible light,
c) after step b), capturing (16) a third image (43) in the scene (110) at the first illumination (50) of the scene (110) at a third point in time (t3), wherein the third image (43) comprises mainly third image information from the scene (110) in the spectral range of visible light,
d) determining (18) at least one reference feature (54) in the scene (110) depicted in the first and third images (41, 43),
e) determining (20) a first change (A) in the field of view using the at least one reference feature (54),
f) registering (22) the second images taking into account at least one second change (B) which results as a first portion of the first change (A), wherein the first portion is a first time difference of the second point in points in time (t2, t2') of two second images (42, 42') to be registered in relation to a second time difference between the third point in time (t3) and the first point in time (t1),
g) processing the registered second images to obtain a result image, and
h) outputting the result image.

2. The method (10') as claimed in claim 1, wherein the method furthermore comprises the following steps:
i) after step b) and before step c), capturing (32) a fourth image (44) in the scene (110) at the first illumination of the scene (110) at a fourth point in time (t4), wherein the fourth image (44) comprises mainly fourth image information from the scene (110) in the spectral range of visible light,
j) after step i) and before step c), capturing (34) a plurality of fifth images (45, 45') in the scene (110) at the second illumination of the scene (110) at a respective fifth point in time (t5, t5'), wherein the fifth images (45, 45') comprise mainly fifth image information from the scene (110) in the spectral range of invisible light,
k) after step e) and before step g), registering (36) the fifth images (45, 45') taking into account at least a third change (B2) which results as a second portion of the first change (A), wherein the second portion is a third time difference of the fifth points in time (t5, t5') of two fifth images (45, 45') to be registered in relation to the second time difference between the third point in time (t3) and the first point in time (t1), and
wherein in step g) the registered second images and the registered fifth images are processed to obtain a result image.

3. An imaging method (10) for imaging a scene (110) during a change in a field of view (112) of an imaging sensor (114) relative to the scene (110), wherein the method (10) comprises the steps of:
a) capturing (12) a first image (41) in the scene (110) at a first illumination (50) of the scene (110) at a first point in time (t1), wherein the first image (41) comprises mainly first image information from the scene (110) in a spectral range of visible light,
b) after step a), capturing (14) a plurality of second images (42, 42') in the scene (110) at a second illumination (52) of the scene (110) at a respective second point in time (t2, t2'), wherein the second images (42, 42') comprise mainly second image information from the scene (110) in a spectral range of invisible light,
b1) after step b), capturing (32) a fourth image (44) in the scene (110) at the first illumination of the scene (110) at a fourth point in time, wherein the fourth image (44) comprises mainly fourth image information from the scene (110) in the spectral range of visible light,
b2) after step b1), capturing (34) a plurality of fifth images in the scene (110) at the second illumination of the scene (110) at a respective fifth point in time, wherein the fifth images comprise mainly fifth image information from the scene (110) in the spectral range of invisible light,
c) after step b2), capturing (16) a third image (43) in the scene (110) at the first illumination (50) of the scene (110) at a third point in time (t3), wherein the third image (43) comprises mainly third image information from the scene (110) in the spectral range of visible light,
d) determining (18') at least one first reference feature (54) in the scene (110) imaged in the first and fourth images (41, 44) and at least one second reference feature (56) in the scene (110) imaged in the fourth (44) and third images (43),
e) determining (20') a first change (A1) in the field of view (112) on the basis of the at least one first reference feature (54) and a further change (A2) in the field of view (112) on the basis of the at least one second reference feature (56),
f) registering (22') the second images (42, 42') taking into account at least one second change (B1) which results as a first portion of the first change (A1), wherein the first portion is a first time difference of the second points in time (t2, t2') of two second images (42, 42') to be registered in relation to a second time difference between the fourth point in time (t4) and the first point in time (t1),
f1) registering (36') the fifth images (45, 45'), taking into account at least one third change (B2) which results as a second portion of the further change (A2), wherein the second portion is a third time difference of the fifth points in time (t5, t5') of two fifth images (45, 45') to be registered in relation to a fourth time difference between the third point in time (t3) and the fourth point in time (t4),
g) processing the registered second images and the registered fifth images to obtain a result image, and
h) outputting the result image.

4. The method (10, 10', 10") according to any preceding claim, wherein a first intensity of the first image (41) and the third image (43) is greater than a second intensity of each of the second images (42, 42').

5. The method (10, 10', 10") according to any preceding claim, wherein the second images (42, 42') comprise mainly second image information from the scene (110) in a near-infrared range.

6. The method (10, 10', 10") according to any preceding claim, wherein the second images (42, 42') are brought into conformity with a reference image.

7. The method (10, 10', 10") according to any preceding claim, wherein the processing (24) of the second registered images comprises the application of computational photography.

8. A system (100) for imaging a scene (110) during a change in a field of view (112) of an imaging sensor (114) relative to the scene (110), the system (100) comprising:
at least one lighting device (102),
at least one imaging device (104),
a processing device (106) configured to cause the system (100) to perform a method according to any preceding claim.

## Revendications

1. Procédé d'imagerie (10) pour imager une scène (110) pendant un changement d'un champ de vision (112) d'un capteur d'image (114) par rapport à la scène (110), le procédé (10) comprenant les étapes consistant à :
a) acquérir (12) une première image (41) dans la scène (110) lors d'un premier éclairage (50) de la scène (110) à un premier instant (t1), la première image (41) comprenant principalement des premières informations d'image de la scène (110) dans un domaine spectral de la lumière visible,
b) après l'étape a), acquérir (14) une pluralité de deuxièmes images (42, 42') dans la scène (110) lors d'un deuxième éclairage (52) de la scène (110) à un deuxième moment respectif (t2, t2'), les deuxièmes images (42, 42') présentant principalement une deuxième information d'image de la scène (110) dans un domaine spectral de la lumière invisible,
c) après l'étape b), acquérir (16) une troisième image (43) dans la scène (110) lors du premier éclairage (50) de la scène (110) à un troisième instant (t3), la troisième image (43) comprenant principalement une troisième information d'image de la scène (110) dans le domaine spectral de la lumière visible,
d) déterminer (18) au moins une caractéristique de référence (54) dans la scène (110), qui est représentée dans la première et la troisième image (41, 43),
e) déterminer (20) une première modification (A) du champ de vision à l'aide de l'au moins une caractéristique de référence (54),
f) enregistrer (22) les deuxièmes images en tenant compte d'au moins une deuxième modification (B) qui est une première part de la première modification (A), la première part étant une première différence de temps des deuxièmes instants (t2, t2') de deux deuxièmes images (42, 42') à enregistrer par rapport à une deuxième différence de temps entre le troisième instant (t3) et le premier instant (t1),
g) traiter les deuxièmes images enregistrées afin d'obtenir une image résultante, et
h) délivrer l'image résultante.

2. Procédé (10') selon la revendication 1, ledit procédé comprenant en outre les étapes consistant à :
i) après l'étape b) et avant l'étape c), acquérir (32) une quatrième image (44) dans la scène (110) lors du premier éclairage de la scène (110) à un quatrième instant (t4), la quatrième image (44) comprenant principalement des quatrièmes informations d'image de la scène (110) dans le domaine spectral de la lumière visible,
j) après l'étape i) et avant l'étape c), acquérir (34) une pluralité de cinquièmes images (45, 45') dans la scène (110) lors du deuxième éclairage de la scène (110) à un cinquième instant respectif (t5, t5'), les cinquièmes images (45, 45') comprenant principalement des cinquièmes informations d'image provenant de la scène (110) dans le domaine spectral de la lumière invisible,
k) après l'étape e) et avant l'étape g), enregistrer (36) les cinquièmes images (45, 45') en tenant compte d'au moins une troisième modification (B2) qui est une deuxième part de la première modification (A), la deuxième part étant une troisième différence de temps des cinquièmes instants (t5, t5') de deux cinquièmes images (45, 45') à enregistrer par rapport à la deuxième différence de temps entre le troisième instant (t3) et le premier instant (t1), et
dans lequel, à l'étape g), les deuxièmes images enregistrées et les cinquièmes images enregistrées sont traitées afin d'obtenir une image résultante.

3. Procédé d'imagerie (10) pour imager une scène (110) pendant un changement d'un champ de vision (112) d'un capteur d'image (114) par rapport à la scène (110), le procédé (10) comprenant les étapes consistant à :
a) acquérir (12) une première image (41) dans la scène (110) lors d'un premier éclairage (50) de la scène (110) à un premier instant (t1), la première image (41) comprenant principalement des premières informations d'image provenant de la scène (110) dans une région spectrale de la lumière visible,
b) après l'étape a), acquérir (14) une pluralité de deuxièmes images (42, 42') dans la scène (110) lors d'un deuxième éclairage (52) de la scène (110) à un deuxième instant respectif (t2, t2'), les deuxièmes images (42, 42') comprenant principalement des deuxièmes informations d'image de la scène (110) dans un domaine spectral de la lumière invisible,
b1) après l'étape b), acquérir (32) une quatrième image (44) dans la scène (110) lors du premier éclairage de la scène (110) à un quatrième instant, la quatrième image (44) comprenant principalement des quatrièmes informations d'image provenant de la scène (110) dans le domaine spectral de la lumière visible,
b2) après l'étape b1), acquérir (34) une pluralité de cinquièmes images dans la scène (110) lors du deuxième éclairage de la scène (110) à un cinquième moment respectif, les cinquièmes images comprenant principalement des cinquièmes informations d'image provenant de la scène (110) dans le domaine spectral de la lumière invisible c) après l'étape b2), acquérir (16) une troisième image (43) dans la scène (110) lors du premier éclairage (50) de la scène (110) à un troisième instant (t3), la troisième image (43) comprenant principalement des troisièmes informations d'image de la scène (110) dans le domaine spectral de la lumière visible,
d) déterminer (18') au moins une première caractéristique de référence (54) dans la scène (110), qui est représentée dans la première et la quatrième image (41, 44), et au moins une deuxième caractéristique de référence (56) dans la scène (110), qui est représentée dans la quatrième (44) et la troisième image (43),
e) déterminer (20') une première modification (A1) du champ de vision (112) à l'aide de l'au moins une première caractéristique de référence (54) et une autre modification (A2) du champ de vision (112) à l'aide de l'au moins une deuxième caractéristique de référence (56),
f) enregistrer (22') les deuxièmes images (42, 42') en tenant compte d'au moins une deuxième modification (B1) qui est une première part de la première modification (A1), la première part étant une première différence de temps des deuxièmes instants (t2, t2') de deux deuxièmes images (42, 42') à enregistrer par rapport à une deuxième différence de temps entre le quatrième instant (t4) et le premier instant (t1),
f1) enregistrer (36') les cinquièmes images (45, 45') en tenant compte d'au moins une troisième modification (B2) qui est une deuxième part de l'autre modification (A2), la deuxième part étant une troisième différence de temps des cinquièmes instants (t5, t5') de deux cinquièmes images (45, 45') à enregistrer par rapport à une quatrième différence de temps entre le troisième instant (t3) et le quatrième instant (t4),
g) traiter les deuxièmes images enregistrées et les cinquièmes images enregistrées afin d'obtenir une image résultante, et
h) délivrer l'image résultante.

4. Procédé (10, 10', 10") selon l'une des revendications précédentes, dans lequel une première intensité de la première image (41) et de la troisième image (43) est respectivement supérieure à une deuxième intensité de chacune des deuxièmes images (42, 42').

5. Procédé (10, 10', 10") selon l'une des revendications précédentes, dans lequel les deuxièmes images (42, 42') comprennent principalement des deuxièmes informations d'image de la scène (110) dans une région proche de l'infrarouge.

6. Procédé (10, 10', 10") selon l'une des revendications précédentes, dans lequel les deuxièmes images (42, 42') sont mises en correspondance avec une image de référence.

7. Procédé (10, 10', 10") selon l'une des revendications précédentes, dans lequel le traitement (24) des secondes images enregistrées comprend l'application de la photographie computationnelle.

8. Système (100) d'imagerie d'une scène (110) pendant une variation d'un champ de vision (112) d'un capteur d'image (114) par rapport à la scène (110), le système (100) comprenant :
au moins un dispositif d'éclairage (102),
au moins un dispositif d'imagerie (104),
un dispositif de traitement (106) adapté pour faire mettre en oeuvre par le système (100) un procédé selon l'une des revendications précédentes.
